# EUROPEAN PATENT APPLICATION

(11) **EP 4 098 656 A1**
(43) Date of publication of application: **07.12.2022**
(21) Application number: 21747750.4
(22) Date of filing: 22.01.2021
(51) Int. Cl.: C07K 7/08, A61K 8/64, A61Q 19/02, A61Q 19/08

(54) **PEPTIDE INHIBITING FORMATION OF SNARE COMPLEX AND USE THEREOF**

(30) Priority: 30.01.2020 KR 20200011344
(71) Applicant: Medytox Inc., Chungcheongbuk-do 28126 (KR)
(72) Inventor: LEE, Jun Ho, Suwon-si Gyeonggi-do 16359 (KR); LEE, Dong Kyu, Suwon-si Gyeonggi-do 16493 (KR); KIM, Dae Hoon, Suwon-si Gyeonggi-do 16220 (KR)
(74) Representative: Brevalex
(86) International application number: PCT/KR2021/000865
(87) International publication number: WO 2021/153946

(57) **Abstract**

Provided are a peptide inhibiting formation of SNARE complexes and a use thereof.

## Description

### TECHNICAL FIELD

The present disclosure is related to a peptide inhibiting formation of a SNARE complex and use thereof.

### BACKGROUND ART

Soluble N-ethylmaleimide-sensitive factor attachment protein receptor (SNARE) complexes are complexes including SNAP-25, syntaxin, and VAMP2, which are involved in the release of neurotransmitters. The neurotransmitter release at synapses requires various proteins that are involved in and behave together with fusion of the neuronal plasma membrane of synaptic vesicles to form synaptic fusion complexes. These proteins are collectively referred to as the SNARE proteins, which include SNAP-25, syntaxin, and synaptobrevin. Synaptobrevin is also referred to as vesicle-associated membrane protein 2 (VAMP2). VAMP2 is located in the synaptic vesicle membrane, whereas SNAP-25 and syntaxin are bonded to the plasma membrane. Calcium excretion causes formation of complexes and pulls the synaptic vesicles close to the plasma membrane, causing the plasma membranes to fuse. By the fusion, the neurotransmitters contained in the vesicles are discharged to the synapse. The neurotransmitters may include acetylcholine or norepinephrine. As a VAMP2-derived peptide, a peptide capable of effectively inhibiting the formation of the SNARE complex is still in demand, but the SNARE protein has a disadvantage in that it cannot effectively act on the skin due to the protective function of the skin.

However, in transporting a drug or a protein for treatment into cells, an attempt is made to deliver a drug into cells using a peptide that acts as a carrier that may transport the target protein into the cells. However, not all proteins form a fusion protein with a protein transport domain and penetrate into cells. For example, there are research results published in papers that a protein bound to a Tat transduction site is introduced into the cell but does not show activity (Sengoku, T. et al. Experimental Neurology 188(2004) 161 - 170, Falnes P.O. et al. Biochemistry 2001 Apr 10;40(14):4349-4358, and Daniele Peroni et al., Neuroscience letters 421(2007) 110-114). That is, fusing the protein transport domain to a protein that is not easily introduceable into cells may not be concluded that the fused protein exhibits significant activity after being introduced into the cell.

### DESCRIPTION OF EMBODIMENTS

### TECHNICAL PROBLEM

According to an aspect of an embodiment of the disclosure, provided is a peptide to which a cell membrane-penetrating peptide is fused to a VAMP2 protein or a fragment thereof.

According to another aspect of an embodiment of the disclosure, provided are a polynucleotide encoding the peptide and a vector and a host cell including the polynucleotide.

According to another of an embodiment of the disclosure, provided is a composition or a kit for skin whitening or reducing wrinkles.

According to another aspect of an embodiment of the disclosure, provided is a method for skin whitening or reducing wrinkles of a subject by administering the peptide to the subject.

According to another aspect of an embodiment of the disclosure, provided is a method of preparing a composition for skin whitening or reducing wrinkles, the method using the peptide.

According to another aspect of an embodiment of the disclosure, provided is a use of the peptide in preparing a skin-whitening agent or a wrinkle-reducing agent.

According to another aspect of an embodiment of the disclosure, provided is the peptide to be used as a skin-whitening agent or a wrinkle-reducing agent.

### SOLUTION TO PROBLEM

According to an aspect of an embodiment of the disclosure, provided is a peptide to which a cell membrane-penetrating peptide is fused to a VAMP2 protein or a fragment thereof.

The VAMP2 protein may consist of the amino acid sequence of SEQ ID NO: 13. The fragment may be an N-terminal fragment of the VAMP2 protein. The fragment may comprise the amino acid sequence of SEQ ID NO: 7 (a sequence of 30^{th} to 43^{th} amino acid residues of SEQ ID NO: 13). The fragment may consist essentially of the amino acid sequence of SEQ ID NO: 7. The fragment may consist of the amino acid sequence of SEQ ID NO: 7. The fragment may comprise the amino acid sequence of SEQ ID NO: 4 (a sequence of 30^{th} to 46^{th} amino acid residues of SEQ ID NO: 13). The fragment may consist essentially of the amino acid sequence of SEQ ID NO: 4. The fragment may consist of the amino acid sequence of SEQ ID NO: 4. The amino acid may be a D- or L-amino acid.

The term "comprising" of the present invention is used synonymously with "containing" or "characterized in that" and does not exclude additional component elements or method steps not mentioned in the composition or method. The term "consist of" refers to excluding additional elements, steps, or components not otherwise mentioned. The term "consist essentially of" is intended to encompass component elements or steps, etc., which, in addition to the described component elements or steps, do not substantially affect their underlying properties.

An N-terminus or C-terminus amino acid of the peptide may be with a reversible chemical modification. The modification may include esterification of the carboxyl groups of glutamic acid and aspartic acid. By the modification, the negative charge of the amino acid may be removed, and hydrophobicity of the amino acid may be increased. The peptides thus modified may have increased bioavailability, including stability and fat solubility, and may allow easy passage through the blood-brain barrier and epithelial tissues. Also, the modification may include amidation of the carboxyl group of the amino acid. The amino acid at the N-terminus of the peptide may be acetylated. The amino acid at the C-terminus of the peptide may be amidated. The modification may be hydrolyzed in the body by intracellular esterase.

The peptide may inhibit the formation of SNARE complexes including SNAP-25, syntaxin, and VAMP2 to inhibit the release of neurotransmitters. The neurotransmitter release at synapses requires various proteins that are involved in and behave together with fusion of the neuronal plasma membrane of synaptic vesicles to form synaptic fusion complexes. These proteins are collectively referred to as the SNARE proteins, which include SNAP-25, syntaxin, and synaptobrevin. Synaptobrevin is also known as VAMP2. VAMP2 is located in the synaptic vesicle membrane, whereas SNAP-25 and syntaxin are bonded to the plasma membrane. Calcium excretion causes formation of complexes and pulls the synaptic vesicles close to the plasma membrane, causing the plasma membranes to fuse. By the fusion, the neurotransmitters contained in the vesicles are discharged to the synapse. The neurotransmitters may include acetylcholine or norepinephrine. The peptide may inhibit the formation of the SNARE complex by mimicking VAMP2, thereby inhibiting the release of neurotransmitters into the synapse. Accordingly, the peptide may abate or ameliorate various symptoms caused by the release of the neurotransmitter into the synapse. The peptide may improve skin whitening or may reduce wrinkles. In addition, the peptide may be used to alleviate or treat neuron-exocytosis mediated symptoms. The symptoms may be spastic ailments. The spastic ailments may be dystonia, strabismus, tics, blepharospasm, or facial scoliosis.

The peptide may be obtained using a conventional solid-phase chemical peptide synthesis method. Also, the peptide may be obtained using a method based on recombinant DNA technology. For example, the method may include introducing a polynucleotide encoding the peptide into an appropriate plasmid or vector, introducing the plasmid or vector into a host cell, culturing the resulting cell so that the peptide is formed in culture, and optionally, purifying the peptide.

The peptide may be fused to a cell membrane-penetrating peptide. The cell membrane-penetrating peptide may increase penetration of the fused peptide through the plasma membrane. The fused peptide may be a peptide in which the C-terminus of the cell membrane-penetrating peptide is fused to the N-terminus of the VAMP2 protein or a fragment thereof. The cell membrane-penetrating peptide may be selected from the group consisting of TD1, IMT-P8, Transkin, VP2-2, RBD-1, SN25-2, STX-1, and TDb-1. The IMT-P8 may consist essentially of the amino acid sequence of SEQ ID NO: 8. The IMT-P8 may consist of the amino acid sequence of SEQ ID NO: 8.

As used herein, the peptide is understood to include a peptide as well as a salt thereof. The salt may be a pharmaceutically or cosmetically acceptable salt of the peptide.

According to another aspect of an embodiment of the disclosure, provided is a composition for skin whitening or reducing wrinkles, the composition including the peptide as an active ingredient.

The composition may further include a pharmaceutically or cosmetically acceptable carrier. The carrier may be a diluent or an excipient. The carrier may be an antioxidant, a stabilizer, a solubilizer, a vitamin, a pigment, or a fragrance. The diluent may be water, buffer, or saline.

Also, the composition may further include a pharmaceutically or cosmetically acceptable adjuvant.

The composition may be a cosmetic or pharmaceutical or food composition.

The composition may be in the form of any preparation, for example, any one formulation selected from an aqueous solution, suspension, emulsion, paste, gel, cream, lotion, powder, soap, surfactant-containing cleanser, oil, powder foundation, emulsion foundation, wax foundation, and spray.

When the formulation is a paste, cream, or gel, one or more of animal oil, vegetable oil, wax, paraffin, starch, tracanth, cellulose derivative, polyethylene glycol, silicone, bentonite, silica, talc, and zinc oxide may be included as a carrier component.

When the formulation is a powder or a spray, the carrier may be one or more of lactose, talc, silica, aluminum hydroxide, calcium silicate, and polyamide powder. When the formulation is a spray, the composition may further include propellants such as chlorofluorohydrocarbons, propane/butane, and dimethyl ether.

When the formulation is a solution or an emulsion, the carrier may include one or more selected from a solvent, a solubilizer, and an emulsifier. The carrier may be, for example, at least one selected from ethanol, isopropanol, ethyl carbonate, ethyl acetate, benzyl alcohol, benzyl benzoate, propylene glycol, 1,3-butylglycol oil, glycerol aliphatic ester, polyethylene glycol, and fatty acid ester of sorbitan.

When the formulation is a suspension, the carrier may be at least one selected from a liquid diluent such as water, ethanol, and propylene glycol; a suspending agent such as ethoxylated isostearyl alcohol, polyoxyethylene sorbitol ester, and polyoxyethylene sorbitan ester; microcrystalline cellulose; aluminum metahydroxide; bentonite; agar; and tragacanth.

An amount of the peptide may be an amount effective to improve skin whitening or to reduce wrinkles. The amount may be, for example, about 0.001 % to about 95 %, about 0.001 % to about 70 %, about 0.001 % to about 50 %, or about 0.01 % to about 50 %, based on the weight of the composition.

According to another aspect of an embodiment of the disclosure, provided is a kit for skin whitening or reducing wrinkles, the kit including the peptide.

The kit may further include at least one selected from the group consisting of a carrier and an adjuvant. Also, the kit may further include an instruction that describes a procedure of using the peptide for skin whitening or reducing wrinkles.

According to another aspect of an embodiment of the disclosure, provided is a method of improving skin whitening and reducing wrinkles of a subject, the method including administering an effective amount of the peptide for improving skin whitening and reducing wrinkles to a subject.

The peptide may be in the form of itself or the composition described above.

The administering of the peptide may be performed by administration through any route. The administration may be oral or parenteral administration. The administration may be performed by applying the peptide to the nasal cavity, mucous membrane, or skin. The subject may be a human or non-human animal, such as a mammal. The method may be a cosmetic method.

According to another aspect of an embodiment of the disclosure, provided is a polynucelotide encoding the peptide.

According to another aspect of an embodiment of the disclosure, provided is a vector including the polynucelotide. The vector may include any one used to deliver the polynucelotide. The vector may include a nucleic acid construct, a plasmid, or a vector derived from a virus. The vector may be an expression vector, for example, an expression vector that may be expressed in a mammal.

According to another aspect of an embodiment of the disclosure, provided is a recombinant host cell including the polynucleotide. The host cell may be a bacterial or animal cell. The animal cell may be a known animal cell such as a Chinese hamster ovary cell (CHO).

According to another aspect of an embodiment of the disclosure, provided is a composition for inhibiting the formation of SNARE complexes including SNAP-25, Syntaxin, and VAMP2, the composition including the peptide as an active ingredient. The composition may further include a carrier or an adjuvant. The composition may be a cosmetic, pharmaceutical, or food composition.

The composition may be used to alleviate or treat neuron-exocytosis mediated symptoms. The symptoms may be spastic ailments. The spastic ailments may be dystonia, strabismus, tics, blepharospasm, or facial scoliosis.

The amount of the peptide and the carrier or adjuvant are the same as described above.

According to another aspect of an embodiment of the disclosure, provided is a kit for inhibiting the formation of SNARE complexes including SNAP-25, Syntaxin, and VAMP2, the kit including the peptide as an active ingredient. The kit may further include at least one selected from a carrier and an adjuvant.

According to another aspect of an embodiment of the disclosure, provided is a method of inhibiting formation of SNARE complexes in a subject, the method including administering the peptide to the subject.

The method may be used to alleviate or treat neuron-exocytosis mediated symptoms. The symptoms may be spastic ailments. The spastic ailments may be dystonia, strabismus, tics, blepharospasm, or facial scoliosis.

The peptide may be in the form of a peptide or a composition including the peptide. An amount of the peptide being administered may be an effective amount to inhibit formation of SNARE complexes in a subject.

According to another aspect of an embodiment of the disclosure, provided is a method of preparing a composition for skin whitening or reducing wrinkles, the method including mixing the peptide with at least one selected from the group consisting of a diluent, an excipient, a carrier, and an adjuvant.

According to another aspect of an embodiment of the disclosure, provided is a use of the peptide in preparing a skin-whitening agent or a wrinkle-reducing agent.

According to another aspect of an embodiment of the disclosure, provided is the peptide to be used as a skin-whitening agent or a wrinkle-reducing agent.

### ADVANTAGEOUS EFFECTS OF DISCLOSURE

A peptide according to an embodiment of the disclosure may be used in skin whitening and reducing wrinkles.

A polynucleotide encoding the peptide, and a vector and a host cell including the polynucleotide, according to an embodiment of the disclosure, may be used to produce the peptide.

A composition or a kit including the peptide according to an embodiment of the disclosure may be used in skin whitening or wrinkle reduction or alleviation or treatment of neuron-exocytosis mediated symptoms.

When a method according to an embodiment of the disclosure is used, skin whitening or wrinkle reduction may occur to a subject, or the neuron-exocytosis mediated symptoms may be alleviated or treated efficiently.

### BRIEF DESCRIPTION OF DRAWINGS

FIG. 1 is a graph illustrating effect of 7 fusion peptides on norepinephrine secretion in PC12 cells;
FIG. 2 is a graph illustrating effect of fusion peptides C2-P4 and P4 on norepinephrine secretion in PC12 cells;
FIG. 3 is a graph showing the results of a cytotoxicity test of C2-P4;
FIG. 4 is a graph showing the results of measuring effect of C2-P4 on skin irritation;
FIG. 5 is a graph showing the results of measuring effect of C2-P4 on a melanin level in cells;
FIG. 6 is a graph showing the results of measuring effect of C2-P4 on a melanin level in a medium; and
FIG. 7 is a graph showing the mRNA expression level of the MITF gene in the presence of C2-P4 and α-MSH.

### BEST MODE

Hereinafter, the disclosure will be described in more detail with reference to the following examples. However, the following examples are provided for illustrative purposes only, and the scope of the disclosure should not be limited thereto in any manner.

Examples and Comparative Examples: Selecting candidate peptide having inhibitory ability of neurotransmitter secretion

Seven peptide fragments were selected from SNAP25, Sytaxin1a, and VAMP2, which are proteins forming the SNARE complex. Table 1 shows seven candidate peptides and peptides for comparison. P1 is the amino acid sequence of Argireline.

**[Table 1]**

| Name | Amino acid sequence | SEQ ID NO: | Note |
|---|---|---|---|
| P1 | EEMQRR | 1 | h-SNAP25 (12-17aa) |
| P2 | DARENEMDENLEQV SGI | 2 | h-SNAP25 (140-156aa) |
| P3 | LSEIETRHSEIIKLENS | 3 | h-Syntaxin1a (192-208aa) |
| P4 | RRLQQTQAQVDEVV DIM | 4 | h-VAMP2 (30-46aa) |
| P5 | DARENEMDENLEQV | 5 | h-SNAP25 (140-153aa) |
| P6 | LSEIETRHSEIIKL | 6 | h-Syntaxin1a (192-205aa) |
| P7 | RRLQQTQAQVDEVV | 7 | h-VAMP2 (30-43aa) |

As shown in Table 1, the peptides of P1 to P7 were all peptides composed of 6 to 17 amino acids derived from amino acid sequences of human SNAP25, Syntaxin 1a, and VAMP2. The numbers in the parentheses represent the locations of the first and last amino acids of the peptide in the amino acid sequence of each of the proteins. Also, fusion peptides having the C-terminus of the cell-penetrating peptide connected to the N-terminus of the peptides of P1 to P7 were prepared. In particular, the cell membrane-penetrating peptide was an IMT-P8 peptide formed of the amino acid sequence of SEQ ID NO: 8. These fusion peptides were named C2-P1, C2-P2, C2-P3, C2-P4, C2-P5, C2-P6, and C2-P7. The peptides of P1 to P7 were chemically synthesized by entrusting to the Lugen Sci (South Korea). Also, the fusion peptides of C2-P1, C2-P2, C2-P3, C2-P4, C2-P5, C2-P6, and C2-P7 were prepared using a chemical synthesis method by entrusting to the Lugen Sci (South Korea) (C2-P4: SEQ ID NO: 14 and C2-P7: SEQ ID NO: 15). Experimental Example 1: Measurement of neurotransmitter inhibitory ability

The 7 fusion peptides were inhibited from forming an SNARE complex to confirm whether they inhibit secretion of neurotransmitters. Norepinephrine was selected as the neurotransmitter.

### (1) Effect of 7 fusion peptides on norepinephrine secretion in neurons

PC12 cells (Korea Cell Line Bank) in an RPMI 1640 medium including 10 % horse serum and 25 µg/mL of antibiotic-antimycotic (Gibco, penicillin 10000 units/mL, streptomycin 10000 µg/mL, Fungizone^{®} (amphotericin B)) in a T-75 flask coated with Martigel were cultured at 37°C for 72 hours. The PC12 cells are a cell line derived from pheochromocytoma arose in the adrenal medulla of rats. Since chromaffin cells, which play a role of catecholamine secretion in the adrenal medulla, are already differentiated and do not divide, culturable PC12 cells are often used to study chromaffin cells.

After culturing, the PC-12 cells were separated from the flask using trypsin-EDTA, and then centrifuged at 1500 rpm for 3 minutes to collect the cells. The collected cells were inoculated in 1 ml of an RPMI 1640 medium containing 10 % horse serum and the antibiotic at a concentration of 1×10⁵ cell/well, and incubated at 37°C for 24 hours in a 48-well plate coated with Matrigel. Thereafter, the peptide of each experimental group, i.e., each of the 7 fusion peptides, was added in a concentration of 20 µM to the RPMI 1640 medium to which nothing was added, and was cultured for 2 hours. Each experimental group was washed twice with a Krebs buffer (118 mM NaCl, 1.2 mM MgSO₄, 2.5 mM CaCl₂, 5 mM KCI, 24 mM NaHCO₂, 2 mM KH₂PO₄, 11 mM dextrose, pH 7.4) and was incubated in a high-potassium ion-containing Krebs buffer (56 mM NaCl, 1.2 mM MgSO₄, 2.5 mM CaCl₂, 68 mM KCI, 24 mM NaHCO₃, 2 mM KH₂PO₄, 11 mM dextrose, pH 7.4) at 37°C for 30 minutes to induce depolarization. An amount of norepinephrine secretion was measured by performing ELISA on the depolarization-induced sample using the Norepinephrine ELISA Kit (Abnova Cat# KA1891). In particular, after washing the depolarization-induced sample, the medium was removed from the wells of the plate, leaving only cells. In order to depolarize the cells, a Krebs buffer solution containing potassium ions was added to the plate to artificially release neurotransmitters, and the neurotransmitters released from the cells into the Krabs buffer were measured through ELISA.

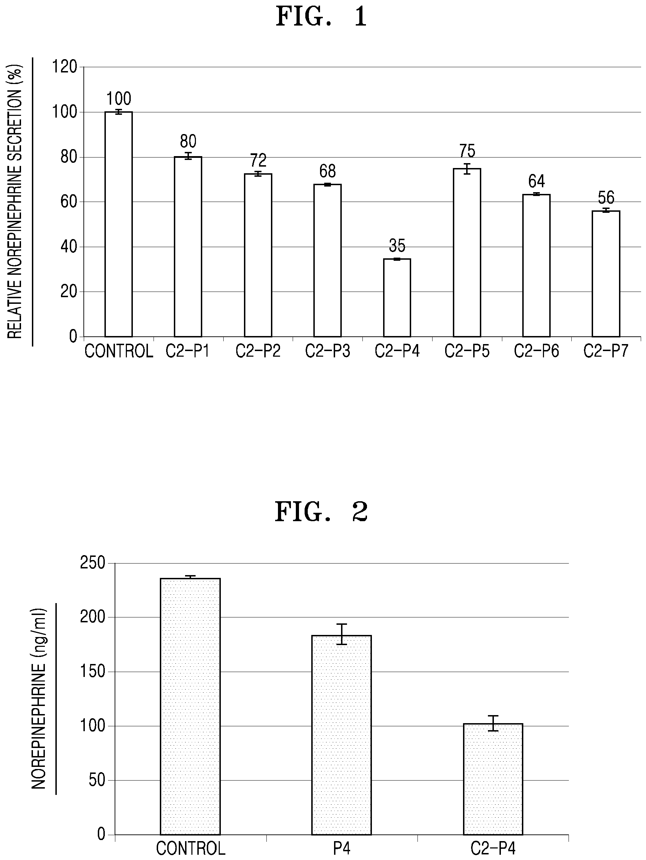

The results are shown in FIG. 1. FIG. 1 is a graph illustrating the effect of the 7 fusion peptides on norepinephrine secretion in PC12 cells. As shown in FIG. 1, it was confirmed that the neurotransmitter secretion rates of the group treated with the C2-P4 peptide and C2-P7 peptide as compared to an untreated group were inhibited to 35 % and 56 %, respectively. The C2-P4 peptide among the 7 fusion peptides most inhibited the neurotransmitter secretion.

### (2) Comparison of ability of inhibiting intracellular neurotransmitter secretion between C2-P4 and P4

PC12 cells (Korea Cell Line Bank) in an RPMI 1640 medium including 10 % horse serum and 25 µg/mL of antibiotic-antimycotic (Gibco, penicillin 10000 units/mL, streptomycin 10000 µg/mL, Fungizone^{®} (amphotericin B)) in a T-75 flask coated with Martigel were cultured at 37°C for 72 hours. The PC12 cells are a cell line derived from pheochromocytoma arose in the adrenal medulla of rats. Since chromaffin cells, which play a role of catecholamine secretion in the adrenal medulla, are already differentiated and do not divide, culturable PC12 cells are often used to study chromaffin cells.

After culturing, the PC-12 cells were separated from the flask using trypsin-EDTA, and then centrifuged at 1500 rpm for 3 minutes to collect the cells. The collected cells were inoculated in 1 ml of an RPMI 1640 medium containing 10 % horse serum and the antibiotic at a concentration of 1×10⁵ cell/well, and incubated at 37°C for 24 hours in a 48-well plate coated with Matrigel. Thereafter, each of the P4 and C2-P4 fusion peptides was added in a concentration of 20 µM to the RPMI 1640 medium to which nothing was added, and was cultured for 2 hours. Each experimental group was washed twice with a Krebs buffer (118 mM NaCl, 1.2 mM MgSO₄, 2.5 mM CaCl₂, 5 mM KCI, 24 mM NaHCO₂, 2 mM KH₂PO₄, 11 mM dextrose, pH 7.4) and was incubated in a high-potassium ion-containing Krebs buffer (56 mM NaCl, 1.2 mM MgSO₄, 2.5 mM CaCl₂, 68 mM KCI, 24 mM NaHCO₃, 2 mM KH₂PO₄, 11 mM dextrose, pH 7.4) at 37°C for 30 minutes to induce depolarization. An amount of norepinephrine secretion was measured by performing ELISA on the depolarization-induced sample using the Norepinephrine ELISA Kit (Abnova Cat# KA1891). In particular, after washing the depolarization-induced sample, the medium was removed from the wells of the plate, leaving only cells. In order to depolarize the cells, a Krebs buffer solution containing potassium ions was added to the plate to artificially release neurotransmitters, and the neurotransmitters released from the cells into the Krabs buffer were measured through ELISA.

The results are shown in FIG. 2. FIG. 2 is a graph illustrating effect of the C2-P4 and P4 fusion peptides on norepinephrine secretion in PC12 cells. As shown in FIG. 2, the neurotransmitter secretion rates of the P4 and C2-P4 peptides as compared to an untreated group were 75 % and 41 %, respectively. That is, the neurotransmitter secretion rate of the C2-P4 in which a cell membrane-penetrating peptide was fused was 34 % lower than that of the P4 to in which a cell-penetrating peptide was not fused.

### Experimental Example 2: Confirmation of toxicity of selected peptide

### (1) Raw material toxicity test: MTT analysis

B16-F10 cells purchased from the Korean Cell Line Bank were inoculated into 100 µL of a DMEM medium containing 10 % FBS at a concentration of 1×10⁴ cells/well in a 96-well plate and cultured for 48 hours. B16-F10 cells are a murine melanoma cell line derived from mice. These cells are adherent and have an epithelial morphology. Thereafter, the C2-P4 peptide was diluted in a DMEM culture medium at concentrations of 12.5 µM, 25 µM, and 50 µM to replace the medium, and the cells were cultured for 48 hours. After centrifuging the plate at 1,000 rpm for 10 minutes, the culture medium solution was removed. Each well was washed twice with PBS, and a 0.5 mg/ml MTT solution diluted in a DMEM medium was added thereto. The cells were cultured in an incubator of 37°C and 5 % CO₂ for 2 hours. After centrifuging the plate at 1,000 rpm for 10 minutes, the MTT solution was removed. Each well was washed twice with PBS, and 150 µL of DMSO was added to dissolve the produced formazan. The plate was placed in a spectrometer (Spectra MAX i3), and absorbance of the sample in each well was measured at 590 nm.

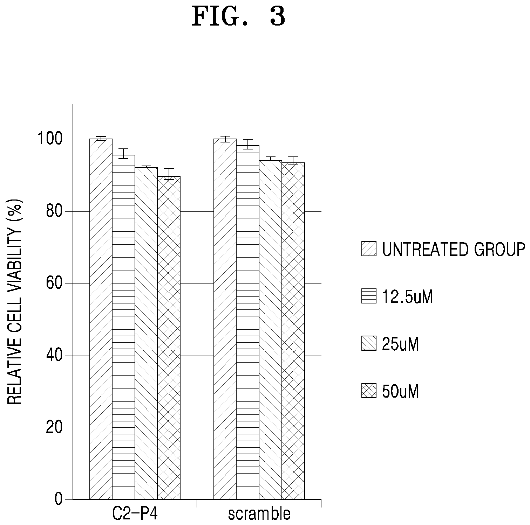

The results are shown in FIG. 3. FIG. 3 is a graph showing the results of a cytotoxicity test of C2-P4. As shown in FIG. 3, C2-P4 showed similar cell viability compared to the negative control, which is a scrambled peptide, in the MTT assay, and there was no cytotoxicity.

### (2) Skin irritation test

1xPBS and 5 % SDS were evenly applied to artificial skin (Neoderm-ED) purchased from Tegoscience (South Korea) to prepare a negative control and a positive control, respectively. An experimental group was evenly coated with 20 µl of 100 ug/ml C2-P4 peptide. After allowing the samples to react at room temperature for 15 minutes, the resultants were each transferred to the wells of a 12-well culture plate and cultured in an incubator of 37°C and 5 % CO₂ for 15 minutes. Once the reaction was completed, the artificial skin was washed twice with PBS and transferred to the wells of a new 12-well culture plate and cultured for 42 hours. Once the culture was completed, 2 ml of 0.3 mg/ml MTT solution was added, and the cells were cultured for 3 hours. The sample in each well of the plate was decolorized by adding 0.04 N HCI-isopropanol, and placed in the Spectra MAX i3, and absorbance of the sample in each well was measured at 580 nm.

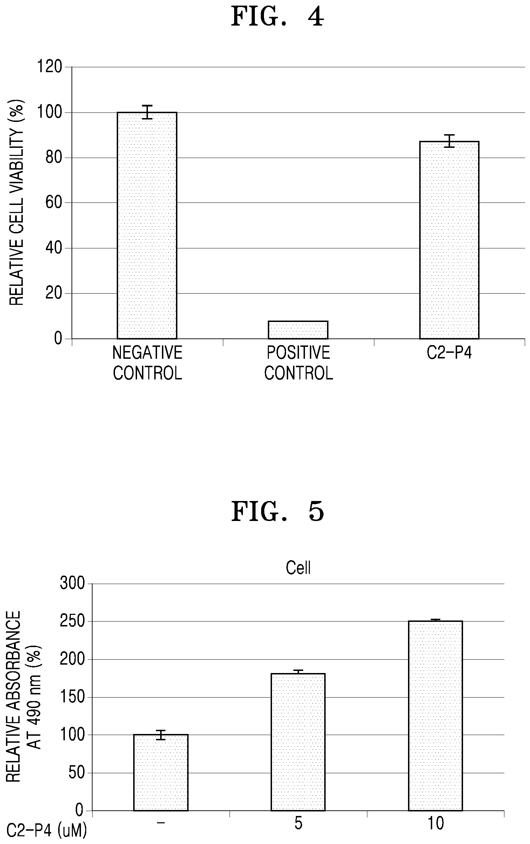

The results are shown in FIG. 4. FIG. 4 is a graph showing the results of measuring effect of C2-P4 on skin irritation. As shown in FIG. 4, the C2-P4 peptide had a cell viability of about 85 % and thus had no irritation. Here, when the cell viability of a sample was 50 % or greater, the sample was determined to have no irritation.

### Experimental Example 3: Confirmation of skin whitening effect of selected peptide

### (1) Measurement of melanin amount in cells after C2-P4 treatment

B16-F10 cells purchased from the Korean Cell Line Bank were inoculated into 1 mL of a DMEM medium containing 10 % FBS at a concentration of 1×10⁴ cells/well in a 24-well plate and cultured in an incubator of 37°C and 5 % CO₂ for 48 hours to form a cell monolayer. Thereafter, the medium was replaced with 10 % FBS and DMEM (Phenol-red free) medium to which 5 µM or 10 µM of C2-P4 peptide was added, and the cells were cultured in an incubator of 37°C and 5 % CO₂ for 48 hours. Once the culture was completed, the experimental group was washed twice with PBS, and the cells were obtained with trypsin-EDTA. The cell monolayer in each well was put into a 1 N NaOH solution having 10 % DMSO added thereto, and incubated at 95°C for 20 minutes to dissolve melanin and the cells, and then put into the Spectra MAX i3, and absorbance of the sample in each well was measured at 490 nm.

The results are shown in FIG. 5. FIG. 5 is a graph showing the results of measuring effect of C2-P4 on a melanin level in cells. As shown in FIG. 5, when the C2-P4 peptide was present in the concentrations of 5 µM and 10 µM, the absorbance was about 180 % and 250 % as compared to the negative control, respectively, and thus the presence of the C2-P4 peptide significantly increased the level of melanin in cells.

### (2) Measurement of melanin amount in medium after C2-P4 treatment

Also, the culture medium from which cells were removed at 48 hours of culture in (1) was put in a 1 N NaOH solution having 10 % DMSO was added thereto, and melanin and the cells were dissolved at 95 °C for 20 minutes, and then absorbance of the sample was measured at 490 nm.

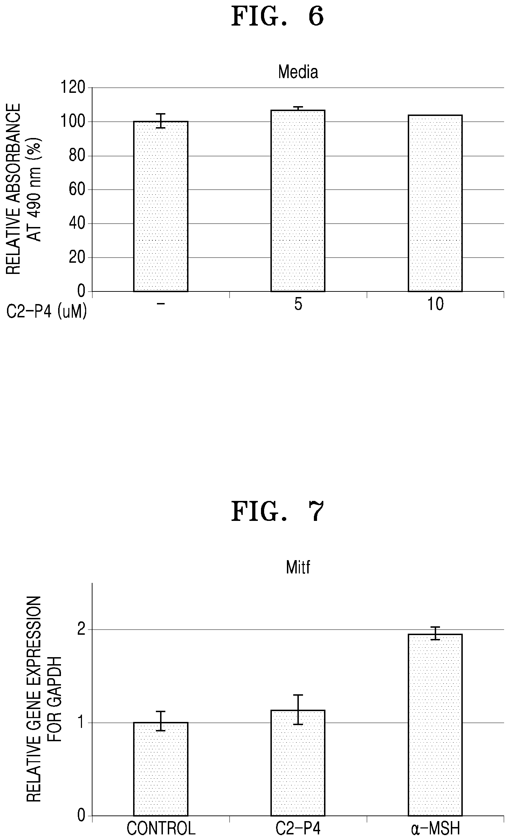

The results are shown in FIG. 6. FIG. 6 is a graph showing the results of measuring effect of C2-P4 on a melanin level in a medium. As shown in FIG. 6, when the C2-P4 peptide was present in the concentrations of 5 µM and 10 µM, the absorbance was about 107 % and 105 % as compared to the negative control, respectively, and thus the level of melanin in the medium did not increase.

According to FIGS. 5 and 6, in the presence of the C2-P4 peptide, the level of melanin in cells increased, while the level of melanin in a medium did not increase, and thus this indicates that secretion of melanin to the outside of the cells is suppressed by the C2-P4 peptide.

### (3) Measurement of MITF gene activity after C2-P4 treatment

B16-F10 cells purchased from the Korean Cell Line Bank were inoculated into 3 mL of a DMEM medium containing 10 % FBS at a concentration of 1×10⁵ cells/well in a 6-well plate and cultured in an incubator of 37°C and 5 % CO₂ for 24 hours to form a cell monolayer. The medium in a negative control was replaced with a DMEM medium, and the medium in peptide-treated experimental groups and α-MSH experimental groups was replaced with a medium containing 10 µM of each of the peptides and 50 nM of α-MSH, respectively, and then the cells were cultured for 48 hours. Each of the experimental groups was washed twice with PBS, the cells were obtained with trypsin-EDTA and centrifuged at 1500 rpm for 5 minutes to separate the cells. RNA was extracted from the separated cells using Trizol, and each 1,000 ng of cDNA was synthesized using reverse transcriptase. Using 100 ng of each of the synthesized cDNAs, the expression levels of GAPDH and α-MSH were confirmed using real-time PCR. The primers used for the real-time PCR were SEQ ID NOs: 9 and 10. Alternatively, the polynucleotide sets of SEQ ID NOs: 11 and 12 were used.

The results are shown in FIG. 7 and Table 2. FIG. 7 is a graph showing the mRNA expression level of the MITF gene in the presence of C2-P4 and α-MSH.

**[Table 2]**

| | Control | C2-P4 | α-MSH |
|---|---|---|---|
| GAPDH | 23.10 | 23.45 | 23.41 |
| | 23.14 | 23.56 | 23.36 |
| | 23.58 | 23.27 | 23.74 |
| MITF | 30.76 | 30.86 | 30.05 |
| | 31.24 | 31.24 | 29.98 |
| | 31.12 | 30.55 | 30.17 |

As shown in FIG. 7 and Table 2, it was confirmed that melanin production by the C2-P4 peptide in B16-F10 cells was not significantly changed, whereas melanin secretion by cells was inhibited by the C2-P4 peptide. Microphthalmia-associated transcription factor (MITF) is known to regulate the expression of various genes essential for normal melanin synthesis in melanocytes in humans. As shown in FIG. 7 and Table 2, the C2-P4 peptide had no significant effect on the expression of the MITF gene, which has a significant effect on melanin formation. This indicates that the C2-P4 peptide causes melanin to accumulate inside the cell by inhibiting secretion of melanin out of the cell rather than increasing melanin production.

## Claims

1. A peptide in which a cell membrane-penetrating peptide is fused to a VAMP2 protein or a fragment thereof.

2. The peptide of claim 1, wherein the fragment comprises the amino acid sequence of SEQ ID NO: 7.

3. The peptide of claim 2, wherein the fragment consistsg of the amino acid sequence of SEQ ID NO: 7.

4. The peptide of claim 2, wherein the fragment comprises the amino acid sequence of SEQ ID NO: 4.

5. The peptide of claim 4, wherein the fragment consists of the amino acid sequence of SEQ ID NO: 4.

6. The peptide of any one of claims 1 to 5, the C-terminus of the cell membrane-penetrating peptide is fused to the N-terminus of the VAMP2 protein or a fragment thereof.

7. The peptide of any one of claims 1 to 6, the cell membrane-penetrating peptide consists ofthe amino acid sequence of SEQ ID NO: 8.

8. The peptide of any one of claims 1 to 7, wherein the peptide consists of the amino acid sequence of SEQ ID NO: 14 or SEQ ID NO: 15.

9. A composition for skin whitening or wrinkles reduction, the composition comprising the peptide of any one of claims 1 to 8 as an active ingredient.

10. The composition of claim 9, further comprising at least one selected from the group consisting of a diluent, an excipient, a carrier, and an adjuvant.

11. A method of whitening skin or reducing wrinkles of a subject, the method comprising administering an effective amount of the peptide of any one of claims 1 to 8 for whitening skin or reducing wrinkles to the subject.

12. The method of claim 11, which is a cosmetic method.

13. A method of preparing a composition for whitening skin or reducing wrinkles, the method comprising mixing the peptide of any one of claims 1 to 8 with at least one selected from a diluent, an excipient, a carrier, and an adjuvant.

14. A use of the peptide of any one of claims 1 to 8 in preparation of a skin-whitening agent or a wrinkle-reducing agent.

15. The peptide of any one of claims 1 to 8 to be used as a skin-whitening agent or a wrinkle-reducing agent.
